# EUROPÄISCHE PATENTSCHRIFT

(11) **EP 1 968 652 B1**
(45) Veröffentlichungstag und Bekanntmachung des Hinweises auf die Patenterteilung: **02.05.2012**
(21) Anmeldenummer: 06840890.5
(22) Anmeldetag: 21.12.2006
(51) Int. Cl.: A61L 2/00

(54) **VERFAHREN ZUR BESTRAHLUNG VON THROMBOZYTENKONZENTRATEN IN FLEXIBLEN BEHÄLTNISSEN MIT ULTRAVIOLETTEM LICHT**
METHOD FOR IRRADIATING THROMBOCYTE CONCENTRATES IN FLEXIBLE CONTAINERS WITH ULTRA-VIOLET LIGHT
PROCÉDÉ POUR EXPOSER DES PRODUITS CONCENTRÉS EN THROMBOCYTES PLACÉS DANS DES CONTENANTS SOUPLES À UNE LUMIÈRE ULTRAVIOLETTE

(30) Priorität: 23.12.2005 DE 102005062410
(43) Veröffentlichungstag der Anmeldung: 17.09.2008
(73) Patentinhaber: Forschungsgemeinschaft der DRK Blutspendedienste E.V., 60528 Frankfurt (DE); Maco Pharma S.A., 59420 Mouvaux (FR)
(72) Erfinder: MOHR, Harald, 30177 Hannover (DE); WALKER, Wolfram, H., 63322 Rödermark (DE)
(74) Vertreter: Müller Schupfner & Partner
(86) Internationale Anmeldenummer: PCT/DE2006/002311
(87) Internationale Veröffentlichungsnummer: WO 2007/076834

(56) Entgegenhaltungen:
- WO-A-03/090795
- WO-A-2005/089816
- US-A- 4 952 812
- US-A- 5 030 200

## Beschreibung

Gegenstand der Erfindung ist ein Verfahren zur Inaktivierung von Pathogenen, wie Bakterien und Viren, und/oder von Leukozyten in Thrombozytenkonzentraten (TKs) durch Bestrahlung mit ultraviolettem Licht.

US 4,952,812 lehrt Thrombozyten - Konzentrate kontaminiert mit Leukozyten mit Wellenlängen von 280 to 320 nm zu bestrahlen. Die weißen Blutkörperchen verlieren hierbei im Wesentlichen Ihre Fähigkeit eine Immunreaktion hervorzurufen. Flache flexible Beutel mit den Thrombozyten - Konzentraten werden hierzu in eine Apparatur gespannt und bestrahlt, um gleichmäßige Schichtdicken auszubilden.

WO 20051089816 A1 offenbart ein Verfahren und eine Vorrichtung zur Behandlung von biologischen Flüssigkeiten in Behältnissen oder Durchflussapparaturen enthaltend photoaktive Stoffe mit elektromagnetischer Strahlung zur Reduktion von Pathogenen. Wellenlängen unter 280 nm werden nicht gelehrt, noch nur geringfügig gefüllte Blutbeutel.

Es ist bekannt, dass die therapeutische Anwendung von Blutpräparaten das Risiko birgt, dass die Empfänger des Blutpräparates mit Viren und Bakterien infiziert werden. Genannt seien z. B. die Viren Hepatitis-B (HBV) und Hepatitis-C (HCV) sowie die Aids-Erreger HIV-1 und HIV-2. Das Risiko besteht immer dann, wenn bei der Herstellung des Präparates kein Schritt zur Inaktivierung bzw. Eliminierung der genannten Pathogene Anwendung findet.

Ultraviolettes (UV-) Licht wird je nach Wellenlänge differenziert. Im Sinne dieser Anmeldung erfolgt folgende Definition: UVA: kleiner 400 bis 320 nm, UVB: kleiner 320 bis 280 nm und UVC kleiner 280 bis 200 nm. Es ist bekannt, dass man durch Bestrahlung mit kurzwelligem ultravioletten (UV-) Licht, d.h. im Wellenbereich unterhalb von ca. 320 nm (UVB- und UVC), sowohl Viren als auch Bakterien inaktivieren kann, etwa in Blutplasma oder in zellulären Blutpräparaten. Oberhalb von 320 nm ist die Energie der Strahlung zu gering, um Mikroorganismen und Viren zu inaktivieren. Gegenüber chemischen, photochemischen und photodynamischen Methoden zur Pathogeninaktivierung besitzt die bloße Bestrahlung mit UV-Licht grundsätzlich den Vorteil für sich allein wirksam zu sein und nicht des Zusatzes reaktiver Chemikalien oder photoaktiver Substanzen zu bedürfen.

Derartige Zusätze bzw. deren Spalt- oder Photoprodukte bedürfen häufig der nachträglichen Entfernung, da sie toxisch oder mutagen sind. Außerdem können sie im behandelten Präparat die Ausprägung neoantigener Strukturen bewirken, wenn sie an Plasmaproteine und Zelloberflächen binden. In der Regel sind derartige Zusätze nicht vollständig entfernbar, zumindest erfordert deren Entfernen zusätzlichen Aufwand. Derartige zusätzliche Arbeitsschritte können überdies die Qualität der sterilisierten Präparate beeinträchtigen. Am effektivsten zur direkten Pathogeninaktivierung ist UVC. Es besitzt allerdings den Nachteil, dass es proteinhaltige Lösungen wie Blutplasma bzw. trübe Suspensionen (z.B. TKs) nur bis zu einer sehr geringen Eindringtiefe durchdringt.

UVC wurde während des zweiten Weltkrieges und noch kurz danach eingesetzt, um Blutplasma und Albuminlösungen zu sterilisieren, vor allem um Hepatitis-Viren zu inaktivieren. Man ging damals so vor, dass die Lösung in einer Durchflussapparatur als dünner Film an einer UVC-Lichtquelle vorbei geleitet wurde. Die Methode erwies sich als nicht genügend sicher und wurde aufgegeben (Kallenbach NR, Cornelius PA, Negus D, et al. Inactivation of viruses by ultraviolet light. Curr Stud Hematol Blood Transfus 1989, 56,70-82).

In heutiger Zeit werden weiterentwickelte nach dem gleichen Prinzip arbeitende Verfahren eingesetzt, um therapeutische Plasmaprotein-Präparationen zu sterilisieren. In allen Fällen ging bzw. geht es darum, größere Volumina zu behandeln, d.h. Plasma-Pools bzw. Proteinlösungen bis zu einigen hundert Litern und sogar mehr (Hart H, Reid K, Hart W. Inactivation of viruses during ultraviolet light treatment of human intravenous immunoglobulin and albumin. Vox Sang 1993;64(2):82-8. und Chin S, Williams B, Gottlieb P, et al. Virucidal short wavelength ultraviolet light treatment of plasma and factor VIII concentrate: protection of proteins by antioxidants; Blood 1995;86(11):4331-6).

Zur Sterilisierung einer Vielzahl einzelner Einheiten von TKs, die aus Blutspenden oder durch maschinelle Apherese gewonnen werden - mit Volumen von allenfalls bis zu einigen Hundert ml - sind die genannten Durchflussapparaturen nicht geeignet. Gerade dies ist aber in der täglichen Praxis einer Blutbank vonnöten.

UVB ist gleichfalls mikrobiozid und viruzid, wenn auch nicht im gleichen Ausmaß wie UVC. Es penetriert proteinhaltige Lösungen und trübe Suspensionen etwas besser als UVC, allerdings lässt sich seine Eindringtiefe, z.B. in Plasma oder TKs, auch nur im Bereich weniger Millimeter feststellen. Erprobt wurde die Bestrahlung mit UVB, um in TKs T-Lymphozyten zu inaktivieren, die beträchtlich UV-sensitiver als Viren oder Bakterien sind. Hierdurch sollte eine Allo-Immunisierung gegen körperfremde HLA-Antigene bei den Empfängern der Präparate verhindert werden, die bewirken kann, dass die Empfänger refraktär gegen weitere Transfusionen von TKs werden (Andreu G, Boccaccio C, Lecrubier C, et al. Ultraviolet irradiation of platelet concentrates: feasibility in transfusion practice. Transfusion 1990;30(5):401-6 und Pamphilon DH. The rationale and use of platelet concentrates irradiated with ultraviolet-B light. Transfus Med Rev 1999;13(4):323-33).

Allerdings setzte sich die Methode nicht durch, weil die fast gleichzeitig entwickelte Leukozytenfiltration eine ähnlich wirksame aber kosten- und vom Arbeitsaufwand her günstigere Alternative darstellt (Leukocyte reduction and ultraviolet B irradiation of platelets to prevent alloimmunization and refractoriness to platelet transfusions. The Trial to Reduce Alloimmunization to Platelets Study Group. N Engl J Med 1997; 337(26):1861-9;).

Es wurde gleichfalls beschrieben, dass Viren in Thrombozyten-Suspensionen durch die Bestrahlung mit monochromatischem UVB-Licht (Wellenlänge 308 nm) inaktiviert werden können. Hierbei wurde ein Excimer-Laser eingesetzt; das Probevolumen betrug einige ml (Prodouz KN, Fratantoni JC, Boone EJ, Bonner RF. Use of laser-UV for inactivation of virus in blood products. Blood 1987;70(2):589-92). Über diesen Maßstab kam man offenbar nicht hinaus. Es ist tatsächlich kein Verfahren aus der Literatur bekannt, mit dem sich Viren bzw. Bakterien in kompletten TKs ausschließlich durch die Bestrahlung mit UV-Licht (d.h. UVB oder UVC) dekontaminieren lassen.

TKs werden durch maschinelle Thrombapherese von einzelnen Spendern gewonnen oder aber auch aus Blutspenden isoliert, wobei die Thrombozyten mehrerer Blutspenden (im Allgemeinen 4-6) gepoolt werden. Das Volumen der hiernach erhältlichen TK liegt im Allgemeinen zwischen ca. 200 und 350 ml. Es werden aber auch TKs aus einzelnen Blutspenden hergestellt, deren Volumen entsprechend kleiner ist (zwischen ca. 40 und 80 ml). Sowohl in Pool- als auch in Apherese-TKs sind die Thrombozyten entweder in Blutplasma oder in speziellen Lagermedien mit einem Restplasmagehalt von ca. 30 bis 40 % suspendiert. Die TKs werden in flachen gasdurchlässigen Kunststoffbeuteln bei 20-24 °C gelagert.

Wünschenswert wäre es, TKs in derartigen Beuteln mit UV-Licht zu sterilisieren. Allerdings besteht hierbei das erwähnte Problem, dass die Präparate fast undurchlässig für UV-Licht sind. Dies soll durch das folgende Rechenbeispiel verdeutlicht werden: sieht man zur Sterilisierung UVB vor und nimmt ein TK-Volumen von ca. 300 ml an, ferner eine Eindringtiefe der UVB-Strahlung von 1 mm sowie die Belichtung von beiden Seiten des Beutels, müsste ein geeigneter Belichtungsbeutel eine Oberfläche von mindestens 1500 cm² haben.

Bei den Pathogenen handelt es sich insbesondere um Viren und/oder Bakterien.

Die Belichtungsbeutel können mit einem Orbitalschüttler, Plattformschüttler, Wippschüttler oder Taumelschüttler geschüttelt werden und werden vorzugsweise während zumindest dreiviertel der gesamten Belichtungsdauer bewegt.

Die Belichtungsbeutel haben typischerweise ein Volumen von bis zu 5000 ml. Wenn die Belichtungsbeutel einseitig aufgelegt sind, ändert sich während und durch das Bewegen bzw. Schütteln die Höhe des Belichtungsbeutels ständig über die gesamte obere Fläche des Belichtungsbeutels, die mit dem Beutelinhalt in Kontakt steht, bezogen auf die Distanz entlang der Flächennormale zwischen Fläche, auf der der Belichtungsbeutel aufliegt, und Schnittpunkt mit der oberen Fläche des Belichtungsbeutels.

Es erscheint schwierig, wenn nicht ausgeschlossen, größere Stückzahlen derartig dimensionierter Beutel routinemäßig zu prozessieren. Das Problem wird noch bedeutend größer, wenn man anstatt mit UVB die TKs mit UVC sterilisieren will, da dessen Eindringtiefe noch viel geringer ist.

Überraschenderweise wurde gefunden, dass obiges Problem durch ein Verfahren gemäß Anspruch 1 gelöst wird. Bevorzugte Ausführungsformen sind Gegenstand der abhängigen Ansprüche oder nachfolgend geschildert.

Nach der vorliegenden Erfindung werden die TKs in ihren Belichtungsbeuteln in geeigneter Weise bewegt. Die Bewegung erfolgt hierbei so heftig, dass sich innerhalb der TKs bereichsweise Schichten ausbilden, die so dünn sind, dass sie von der UV-Strahlung durchdrungen werden können. Die Bewegung muss gleichzeitig so sein, dass die TK-Suspensionen in den Beuteln wirksam vermischt werden. Beides ist zu realisieren, wenn folgende Voraussetzungen gegeben sind:
1. Die Belichtungsbeutel sind hochflexibel, und sie werden während der Belichtung nicht fixiert, z.B. zwischen Quarzplatten eingeklemmt. Sie passen sich somit jeder Gestaltsänderung der TK-Suspension an, die sich ergibt, wenn die Beutel bewegt werden.
2. Die Bewegung der Beutel erfolgt entweder horizontal (linear in Hin- und Her-Richtung bzw. kreis- oder ellipsenfömig) oder aber vertikal (gewippt).
3. Die Belichtungsbeutel sind zu maximal 30 % , insbesondere zu maximal 20 % ihres maximalen Füllvolumens gefüllt.

Auf alle Fälle sollte die Umkehrung der Bewegungsrichtung so abrupt sein, dass der größere Teil der TK-Suspension sich infolge seiner Trägheit weiter in die ursprüngliche Richtung bewegt und somit der zurückbleibende Rest eine dünne Schicht ausbilden kann, die für die UV-Strahlung durchdringbar ist. In Verbindung mit der ständigen Durchmischung infolge der Bewegung der Beutel während der Belichtung wird letztendlich das gesamte TK (und die darin enthaltenen Viren und/oder Bakterien) der UV-Strahlung ausgesetzt. Somit werden die TKs sterilisiert.

Die Belichtungsbeutel sind aus UV- transparentem Kunststoffmaterial gefertigt. Geeignete Kunststoffe sind z.B. Ethylenvinylacetat und Polyolefine mit Folienstärken von 1 mm und weniger, insbesondere Folienstärken kleiner 0,5 mm. Die Belichtungsbeutel sind flächig ausgebildet und weisen vorzugsweise keine Absorptionsmaxima im Bereich von 200 bis 320 nm auf. Die Belichtungsbeutel sind im liegenden gefüllten Zustand nur einige mm dick, z.B. kleiner 10 mm und insbesondere 5 mm, vorzugsweise sogar kleiner 3 mm und sind bestimmt, Probenvolumina von z.B. bis zu 200 oder bis zu 300 ml aufnehmen. Das maximale Fassungsvermögen (Volumen) des Belichtungsbeutels ist aber um mindestens Faktor 3, i.d.R. um mindestens 5 mal größer, vorzugsweise mindestens 10 oder sogar mindestens 20 mal größer als das tatsächliche in ihm enthaltene zu behandelnde Probenvolumen.

### Experimentelle Untersuchungen

Die beschriebenen Versuche illustrieren die Wirksamkeit des Verfahrens und sind nicht auf die Inaktivierung der nachgenannten Bakterien und/oder Viren beschränkt. Es besteht auch keine Einschränkung auf "Random Donor"-TKs, die bei den beschriebenen Versuchen verwendet wurden, und das erfiridungsgemäße Verfahren ist auch auf Thrombapheresepräparate anwendbar. Alle Versuche wurden drei bis sechs Mal durchgeführt. Die angebenen Ergebnisse stellen jeweils die Mittelwerte ± Standardabweichung dar.

### Thromboytenkonzentrate

Die TKs wurden aus Pools von jeweils 5 Buffy Coats hergestellt, die ihrerseits aus regulären Blutspenden stammten. Die TKs hatten ein Volumen von ca. 300 bis 350 ml; die Thrombozytenkonzentration betrug ca. 10⁹/ml. Die Thrombozyten waren im Lagermedium SSP+ (Produkt der Firma MacoPharma) suspendiert. Der Restplasmagehalt betrug ca. 30 bis 40%.

### Bakteriologische Untersuchungen

Bei den Inaktivierungsversuchen wurden folgende Bakterienstämme eingesetzt:
*Staphylococcus (S.) epidermis*
*Staphylococcus (S.) aureus*
*Bacillus (B.) cereus*
*Klebsiella (K.) pneumoniae.*

Bakterienkonzentrationen wurden mittels eines Koloniebildungsassays bestimmt und werden als Colony forming units (CFU)/ml ausgedrückt. Bei den Versuchen zur Inaktivierung von Bakterien wurden komplette TKs bzw. TK-Aliquots mit 10⁴ bis 10⁵ CFU/ml eines der angegebenen Spezies gespikt und dann mit UV-Licht bestrahlt.

### Virologische Untersuchungen

TK-Aliquots wurden mit Suid Herpesvirus (SHV-1, Pseudorabiesvirus, Stamm Aujeszky) bzw. Vesicular Stomatitisvirus (VSV, Stamm Indiana) gespikt. Virustiter wurden mittels CPE-Assay (CPE = cytopathischer Effekt) bestimmt. Sie werden als TCID₅₀ (TCID = Tissue culture infective dose) angegeben. Als Indikatorzellen dienten Vero-Zellen. Die initiale Viruskonzentration bei den durchgeführten Versuchen betrug ca. 10⁵ bis 10⁷ TCID₅₀.

### Bel ichtungsanlagen

Die eine der beiden verwendeten Belichtungsanlagen war mit Röhren ausgestattet, die UVB-Licht emittierten. Die Bestrahlung erfolgte von beiden Seiten der aufgelegten Belichtungsbeutel, d.h. von oben und von unten. Die Belichtungsanlage war mit einer Schüttelvorrichtung versehen, die horizontale Hin- und Herbewegungen mit einer Frequenz von 60 Richtungswechseln/min durchführte. Eine zweite Belichtungsanlage war gleichfalls mit Röhren ausgestattet, die UVB-Licht emittierten. Die Bestrahlung erfolgte ebenfalls von beiden Seiten. Eine dritte Anlage (gleicher Bauart wie die zweite) war mit Röhren versehen, die UVC-Licht (Wellenlänge: 254 nm) emittierten. Beide Anlagen konnten mit 2 unterschiedlichen Schüttelvorrichtungen versehen werden: einem Horizontalschüttler, der ellipsoide Hin- und Herbewegungen machte, und einem Wippschüttler.

### Belichtungsbeutel

Die verwendeten Belichtungsbeutel bestanden aus Ethylvinylacetat (EVA), das UVdurchlässig ist. Es wurden zwei Beutelgrößen verwendet:
1. 14,5 x 18,5 cm (äußere Beutelfläche ca. 268 cm²)
2. 22,5 x 38 cm (äußere Beutelfläche ca. 855 cm²)

Bei den Versuchen mit den kleinen EVA-Beuteln betrug das Probevolumen 80 ml, bei denen mit den großen Beuteln ca. 300-350 ml (es wurden komplette TKs behandelt).

### Versuchsbeispiel 1:

### Inaktivierung von S. epidermidis durch UVB, mit und ohne freie Bewegung der Thrombozytensuspension während des Schüttelns

Bei dem Versuch betrug das Probevolumen 80 ml. Die freie Bewegbarkeit der Thrombozytensuspension während des Schüttelns und damit die Ausbildung einer dünnen Schicht wurde in einer Probe dadurch verhindert, dass die Belichtungsbeutel fest zwischen zwei Quarzplatten eingeklemmt wurden. Die resultierende Schichtdicke betrug ca. 3 mm. Bei der zweiten Probe wurde der Abstand zwischen den Quarzplatten derart vergrößert, dass sich die Thrombozytensuspension während des Schüttelns weitgehend frei bewegen konnte. Die beiden Proben wurden mit 1 J/cm² bestrahlt.

Wie die Tab 1 zeigt, wurde der Bakterientiter in den fixierten Proben um ca. 2 log₁₀ reduziert, in den lose platzierten dagegen um mehr als 4 log₁₀.

**Tab. 1**

| Probenbezeichnung | Bakterientiter (log₁₀CFU/ml) |
|---|---|
| Unbehandelte Kontrolle | 4,1±0,03 |
| Fest eingespannte Probe | 1,4±1,29 |
| Lose platzierte Probe | -0,40+0,35 |

### Versuchsbeispiel 2:

### Inaktivierung von S. epidermidis durch UVB in kompletten TKs, lose bzw. fest eingespannte Belichtungsbeutel, mit Schütteln

Das TK-Volumen bei diesem Versuch betrug 330 ml, die durchschnittliche Schichtdicke in großen EVA-Beuteln demnach ca. 3,9 mm. Die TKs wurden unter folgenden Bedingungen mit 3 UVB-Dosen (0,8, 1,0 und 1,2 J/cm²) bestrahlt:
1. ohne Schütteln, lose zwischen Quarzplatten platziert
2. mit Schütteln, zwischen Quarzplatten gepresst.

Wie die Versuchsergebnisse zeigen (Tab. 2), wurde in den TKs, die während des Schüttelns fest eingespannt waren, durch die UVB-Behandlung der Bakterientiter um bis zu etwa 2 log₁₀ reduziert, demgegenüber in den lose platzierten Proben dosisabhängig um etwa 3,4 bis zu mehr als 4 log₁₀.

**Tab.2**

| Probenplatzierung | UVB-Dosis (J/cm²) | Bakterien-Titer (log₁₀CFU/ml) |
|---|---|---|
| geschüttelt, fest eingespannt | 0 | 4,11±0,00 |
| geschüttelt, fest eingespannt | 0,8 | 2,14±0,48 |
| geschüttelt, fest eingespannt | 1,0 | 1,95±0,03 |
| geschüttelt, fest eingespannt | 1,2 | 1,99±0,03 |
| geschüttelt, lose | 0 | 4,19±0,11 |
| geschüttelt, lose | 0,8 | 0,77±0,69 |
| geschüttelt, lose | 1,0 | -0,14±0,05 |
| geschüttelt, lose | 1,2 | -0,40±0,05 |

### Versuchsbeispiel 3:

### Inaktivierung weiterer Bakterien in frei beweglichen bzw. fixierten TK-Aliquots durch UVB

Aus den ersten beiden Versuchsbeispielen geht hervor, dass *S*. *epidermidis* in TKs unter der Voraussetzung, dass die TK-Suspension sich während der UV-Bestrahlung frei bewegen kann, effektiv inaktiviert wird. Beim folgenden Versuch wurden folgende zusätzliche Bakterienstämme getestet: *S*. *aureus, B. cereus* und *K. pneumoniae.*

Die Bedingungen waren die gleichen wie im Versuchsbeispiel 1 beschrieben. In allen drei Fällen ein ähnliches Ergebnis wie mit S. *epidermidis:* in den lose platzierten TK-Proben wurden die Bakterien um ca. 3,9 bis 4,25 log₁₀ inaktiviert, während die Titer in den fixierten Proben nur um ca. 2 bis 3,4 log₁₀ reduziert wurden (Tab. 3).

**Tab. 3**

| Bakterienstamm | Probenbezeichnung | Bakterientiter (log₁₀CFU/ml) |
|---|---|---|
| *S. aureus* | unbehandelte Kontrolle | 4,88±0,00 |
| *S. aureus* | fest eingespannt, geschüttelt | 1,49+1,30 |
| *S. aureus* | lose platziert, geschüttelt | 0,97±0,86 |
| *B. cereus* | unbehandelte Kontrolle | 4,99±0,09 |
| *B. cereus* | fest eingespannt, geschüttelt | 2,99±0,13 |
| *B. cereus* | lose platziert, geschüttelt | 0,74±0,68 |
| *K. pneumoniae* | unbehandelte Kontrolle | 4,94±0,08 |
| *K. pneumoniae* | fest eingespannt, geschüttelt | 2,34+0,24 |
| *K. pneumoniae* | lose platziert, geschüttelt | 1,00±0,89 |

### Versuchsbeispiel 4:

### Inaktivierung von S. epidermidis in TK-Aliquots durch UVB unter verschiedenen Schüttelbedingungen

Es wurde untersucht, ob die Inaktivierung von *S*. *epidermidis* in lose platzierten TK-Aliquots auch dann gesteigert wird, wenn andere als der bei den Versuchen 1 bis 3 verwendete Horizontalschüttler eingesetzt werden, der wie erwähnt Hin- und Herbewegungen macht. Bei den folgenden Versuchen wurde ein Orbitalschüttler benutzt, der eine kreisförmige Bewegung durchführte (Radius: 3 cm, Drehzahl: 50/min), ferner eine Wippe mit 50 Auf- und Abbewegungen pro Minute. Wiederum war eine der beiden Proben (80 ml) fest zwischen Quarzplatten eingespannt, die andere war lose platziert. Wie aus den in der Tab. 4 gezeigten Ergebnissen hervorgeht, war diesmal das Ausmaß der Bakterieninaktivierung bei den lose platzierten TK-Proben um 3 bis 4 log₁₀ höher als bei den fest eingespannten.

**Tab. 4**

| Schüttler | Probenbezeichnung | Bakterientiter (log₁₀CFU/ml) |
|---|---|---|
| --- | unbehandelte Kontrolle | 4,94±0,16 |
| Orbital | fest eingespannt | 4,23±0,00 |
| Orbital | lose platziert | 0,50±0,39 |
| Wippe | fest eingespannt | 4,02±0,17 |
| Wippe | lose platziert | 0,87±0,78 |

### Versuchsbeispiel 5:

### Inaktivierung von Suid Herpesviren durch UVB, ohne bzw. mit freier Bewegung der TK-Aliquots während des Schüttelns

Um zu prüfen, ob die Steigerung der Inaktivierung von Pathogenen in TKs, die während der Bestrahlung mit UV-Licht nicht fixiert werden, nicht nur Bakterien, sondern auch Viren betrifft, wurde folgender Versuch durchgeführt: TK-Aliquots von 80 ml wurden mit Suid Herpesviren (SHV-1) gespikt und wie im Versuchsbeispiel 1 beschrieben, mit UVB behandelt. In den frei platzierten Proben wurde der Virustiter um fast 4 log₁₀ reduziert, in den fest eingespannten dagegen nur um ca, 3 log₁₀. Dies bestätigt, dass unter den genannten Bedingungen auch die Inaktivierung von Viren deutlich verbessert wird.

**Tab.5**

| Probenbezeichnung | Virustiter (Log₁₀TCID₅₀) |
|---|---|
| unbehandelte Kontrolle | 4,4±0,2 |
| fest eingespannte Probe | 1,41±0,18 |
| lose platzierte Probe | 0,56±0,15 |

### Versuchsbeispiel 6:

### Inaktivierung von Vesicular Stomatitisviren durch UVB, ohne bzw. mit freier Bewegung der TK-Aliquots während des Schüttelns

Die Durchführung erfolgte wie im Versuchsbeispiel 5 beschrieben, nur dass die TK-Aliquots statt mit SHV-1 mit VSV gespikt wurden. Wiederum war das Ausmaß der Virus-Inaktivierung in den lose platzierten Proben mit mehr als 6,46 log₁₀ stärker als in den Vergleichsproben, die während der Belichtung zwischen Quarzplatten fixiert waren (Tab. 6). Allerdings fällt auf, dass auch in diesen der Virustiter relativ stark reduziert wurde (um ca. 5,4 log₁₀). Offenbar ist VSV UV-sensitiver als SHV-1.

**Tab.6**

| Probenbezeichnung | Virustiter (Log₁₀TCID₅₀) |
|---|---|
| unbehandelte Kontrolle | 6,7±1,05 |
| fest eingespannte Probe | 1,29±1,05 |
| lose platzierte Probe | ≤ 0,24±0,00 |

### Versuchsbeispiel 7:

### Inaktivierung von S. epidermidis durch UVC, ohne bzw. mit freier Bewegung der TK-Aliquots während des Schüttelns

Bei diesem Versuch erfolgte die Bestrahlung mit UVC anstelle von UVB. Die UV-Dosis betrug 0,3 J/cm² (Belichtungszeit: 60 sec). Ansonsten waren die Bedingungen wie im Versuchsbeispiel 1 beschrieben. Wie aus der Tab. 7 hervorgeht, wurde der Bakterientiter in den fixierten Proben um nur ca. 1 log₁₀ reduziert. Dies reflektiert offenbar die geringe Eindringtiefe von UVC in die Thrombozytensuspension. In den lose platzierten Proben waren dagegen keine Bakterien mehr nachweisbar; der Inaktivierungsfaktor war größer als 4 log₁₀.

**Tab.7**

| Probenbezeichnung | Bakterientiter (log₁₀CFU/ml) |
|---|---|
| unbehandelte Kontrolle | 4,08±0,04 |
| fest eingespannte Probe | 2,99±0,13 |
| lose platzierte Probe | ≤ 0,24 |

### Versuchsbeispiel 8:

### Inaktivierung von VSV durch UVC, ohne bzw. mit freier Bewegung der TK-Aliquots während des Schüttelns

Wie im Versuchsbeispiel 6 wurde als Testvirus VSV eingesetzt. Die Bedingungen waren die gleichen wie im Versuchsbeispiel 7. Die in der Tab. 8 gezeigten Ergebnisse zeigen, dass auch in diesem Fall das Ausmaß der Pathogeninaktivierung in den lose platzierten Proben viel ausgeprägter war als in den fixierten: während der Virustiter in diesen nur um etwa 1,5 log₁₀ reduziert wurde, waren es in den nicht fixierten Proben etwa 6,2 log₁₀.

**Tab. 8**

| Probenbezeichnung | Virustiter (Log₁₀TCID₅₀) |
|---|---|
| unbehandelte Kontrolle | 6,92±0,22 |
| fest eingespannte Probe | 5,47±0,21 |
| lose platzierte Probe | 0,74±0,76 |

## Patentansprüche

1. Verfahren zur Inaktivierung von Pathogenen und/oder Leukozyten in Thrombozyten-Konzentraten umfassend die Schritte:
- Bereitstellen von aus Spenderblut und/oder durch maschinelle Apherese gewonnener sich jeweils in flexiblen UV-durchlässigen flächigen Belichtungsbeuteln befindenden Thrombozyten-Konzentraten, die aus einer Vielzahl von einzeln handhabbaren und getrennt aufbewahrten Einheiten bestehen (TKs),
- Aussetzen der TKs einer Bestrahlung mit ultraviolettem (UV-) Licht, **dadurch gekennzeichnet, dass**
- die Belichtungsbeutel zu weniger als 30 % des maximalen Füllvolumens der Belichtungsbeutel gefüllt und
- die Belichtungsbeutel während der Bestrahlung mit UV-Licht bewegt werden, so dass der Betichtungsbeutel-Inhalt umgewälzt wird und sich durch die Bewegung Zonen wechselnder Schichtdicke ausbilden, wobei das eingesetzte UV-Licht UVC-Strahlung von kleiner 280 nm bis 200 nm umfasst.

2. Verfahren gemäß einem der vorhergehenden Ansprüche, **dadurch gekennzeichnet, dass** die Belichtungsbeutel durch die Bewegung die Zonen bestrahlte Bereiche aufweisen, für die sich regelmäßig zeitweise Schichtdicken unter 1 mm ergeben, wobei die Belichtungsbeutel durch Schütteln, Wippen oder durch Rotation bewegt werden.

3. Verfahren gemäß einem der vorhergehenden Ansprüche, **dadurch gekennzeichnet, dass** die Bewegung, insbesondere die Amplitude der Bewegung, so erfolgt, dass sich innerhalb der TKs bestrahlte Bereiche ausbilden, in der die Schichtdicken regelmäßig zeitweise geringer als 0,5 mm ist, wobei die Belichtungsbeutel durch Schütteln, Wippen oder durch Rotation bewegt werden.

4. Verfahren gemäß einem der vorhergehenden Ansprüche, **dadurch gekennzeichnet, dass** die Belichtungsbeutel eine Unterseite und eine Oberseite aufweisen und die Summe der Flächen der Unterseite und Oberseite, die mit dem Beutelinhalt in Kontakt steht bzw. stehen kann, mehr als 90 Flächenprozent, vorzugsweise mehr als 99 Flächenprozent, der inneren Gesamtoberfläche des Beutelinhalts ausmacht.

5. Verfahren gemäß einem der vorhergehenden Ansprüche, **dadurch gekennzeichnet, dass** die Bestrahlung weiterhin UVB-Strahlung von kleiner als 320 nm bis 280 nm umfasst und vorzugsweise ausschließlich aus Bestrahlung mit Wellenlängen von kleiner 280 nm bis 200 nm und kleiner 320 nm bis 280 nm besteht.

6. Verfahren gemäß einem der vorhergehenden Ansprüche, **dadurch gekennzeichnet, dass** jede Einheit aus Proben von bis zu maximal 8 Spendern, vorzugsweise von bis zu maximal 6 Spendern hergestellt ist und insbesondere jede Einheit von einem Spender stammt.

7. Verfahren gemäß einem der vorhergehenden Ansprüche, **dadurch gekennzeichnet, dass** das TK zumindest 1x10⁸ Thrombozyten pro ml enthält, vorzugsweise zumindest 5x10⁸ Thrombozyten pro ml.

8. Verfahren gemäß Anspruch 5, **dadurch gekennzeichnet, dass** die Bestrahlung mit UVB mit einer Lichtenergie von 0,3 bis 5 J/cm², vorzugsweise 0,5 bis 2,5 J/cm² erfolgt.

9. Verfahren gemäß einem der vorhergehenden Ansprüche, **dadurch gekennzeichnet, dass** die Bestrahlung mit UVC mit einer Lichtenergie von 0,01 bis 2 J/cm², vorzugsweise 0,1 bis 1 J/cm² erfolgt.

10. Verfahren gemäß einem der vorhergehenden Ansprüche, **dadurch gekennzeichnet, dass** die TKs
(a) Plasma und ein geeignetes Lagerungsmedium enthalten, wobei der Plasmagehalt vorzugsweise größer 20 Gew.% beträgt und/oder
(b) ein gepuffertes wässriges Lagerungsmedium enthalten.

11. Verfahren gemäß einem der vorhergehenden Ansprüche, **dadurch gekennzeichnet, dass** die TKs frei von photoaktiven Substanzen sind.

12. Verfahren gemäß einem der vorhergehenden Ansprüche, **dadurch gekennzeichnet, dass** die Belichtungsbeutel in einer Apparatur, in der die Belichtungsbeutel bewegt und bestrahlt werden, beweglich gehaltert sind und insbesondere nicht zwischen zwei Flächen, z.B. UV durchlässige Glas- oder Kunststoffplatten, eingespannt sind.

13. Verfahren gemäß zumindest einem der vorhergehenden Ansprüche, **dadurch gekennzeichnet, dass** die Belichtungsbeutel eine mittlere Füllhöhe von weniger als 10 mm, bevorzugt weniger als 5 mm, aufweisen und durch die Bewegung ständig Wellentäler erzeugt werden, die Schichtdicken von kleiner der Hälfte der mittleren Füllhöhe, vorzugsweise Schichtdicken von kleiner 1 mm oder sogar kleiner 0,1 mm, besitzen.

14. Verfahren gemäß zumindest einem der vorhergehenden Ansprüche, **dadurch gekennzeichnet, dass** die Belichtungsbeutel während der Bestrahlung ständig mit einer Amplitude von 0,2 bis 8 cm zumindest in der x-Richtung und ggfs. auch in der γ-Richtung (γ-Richtung rechtwinklig zur x-Richtung) bewegt werden und unabhängig hiervon die Frequenz der Richtungsänderung der Schüttelbewegung 0,5 bis 10 Hz beträgt.

15. Verfahren gemäß zumindest einem der vorhergehenden Ansprüche, **dadurch gekennzeichnet, dass** die Belichtungsbeutel während der Bestrahlung zu maximal 20 % ihres maximalen Füllvolumens befüllt sind.

## Claims

1. A method for inactivating pathogens and/or leucocytes in thrombocyte concentrates comprising the steps of:
- providing thrombocyte concentrates obtained from donor blood and/or by machine aphaeresis, respectively located in flexible UV-permeable flat exposure bags, which thrombocyte concentrates consist of a multitude of individually manageable and separately stored units (TCs),
- exposing said TCs to irradiation with ultra-violet (UV) light,
**characterized in that**
- said exposure bags are filled at less than 30 % of the maximum filling volume of said exposure bags, and
- said exposure bags are moved during said irradiation with UV light, such that the exposure bag contents is circulated and with the movement, zones of varying layer thickness are formed, wherein said UV light used comprises UVC radiation of less than 280 nm to 200 nm.

2. The method according to any of the preceding claims, **characterized in that**, due to said movement, said exposure bags have zones of radiated areas, for which, at regular intervals, layer thicknesses of less than 1 mm result at times, wherein said exposure bags are moved by shaking, rocking or by rotation.

3. The method according to any of the preceding claims, **characterized in that** said movement, in particular the amplitude of said movement, takes place such that radiated areas are formed within the TCs, in which, at regular intervals, the layer thicknesses are less than 0.5 mm at times, wherein said exposure bags are moved by shaking, rocking or by rotation.

4. The method according to any of the preceding claims, **characterized in that** said exposure bags have a bottom side and a top side, and the sum of the areas of said bottom side and top side, which are or can be in contact, respectively, with the bag contents, constitutes more than 90 area percent, preferably more than 99 area percent of the overall inner area of the bag contents.

5. The method according to any of the preceding claims, **characterized in that** said irradiation furthermore comprises UVB radiation of less than 320 nm to 280 nm, and preferably exclusively consists of irradiation with wavelengths of less than 280 nm to 200 nm and less than 320 nm to 280 nm.

6. The method according to any of the preceding claims, **characterized in that** each unit is manufactured from samples of up to max. 8 donors, preferably up to max. 6 donors, and in particular each unit originates from one donor.

7. The method according to any of the preceding claims, **characterized in that** said TC contains at least 1x10⁸ thrombocytes per ml, preferably at least 5x10⁸ thrombocytes per ml.

8. The method according to claim 5, **characterized in that** said irradiation with UVB takes place with a light energy of 0.3 to 5 J/cm², preferably 0.5 to 2.5 J/cm²_{.}

9. The method according to any of the preceding claims, **characterized in that** said irradiation with UVC takes place with a light energy of 0.01 to 2 J/cm², preferably 0.1 to 1 J/cm².

10. The method according to any of the preceding claims, **characterized in that** said TCs
(a) contain plasma and a suitable storage medium, wherein the plasma content preferably is higher than 20 wt/%, and/or
(b) contain a buffered aqueous storage medium.

11. The method according to any of the preceding claims, **characterized in that** said TCs are free from photoactive substances.

12. The method according to any of the preceding claims, **characterized in that** said exposure bags are movably held in an apparatus, in which said exposure bags are moved and radiated, and in particular are not restrained between two surfaces, e.g. UV-permeable glass or plastic plates.

13. The method according to at least one of the preceding claims, **characterized in that** said exposure bags have an average filling level of less than 10 mm, preferably less than 5 mm, and with said movement, wave troughs are constantly created, which have layer thicknesses of less than half the average filling level, preferably layer thicknesses of less than 1 mm or even less than 0.1 mm.

14. The method according to at least one of the preceding claims, **characterized in that** during said irradiation said exposure bags are constantly moved with an amplitude of 0.2 to 8 cm at least in the x-direction and possibly also in the y-direction (y-direction perpendicular to the x-direction), and independent hereof, the frequency of the change in direction of the shaking movement is 0.5 to 10 Hz.

15. The method according to at least one of the preceding claims, **characterized in that** during said irradiation said exposure bags are filled at max. 20 % of their maximum filling volume.

## Revendications

1. Procédé pour inactiver des pathogènes et/ou des leucocytes dans des concentrés thrombocytaires, comprenant les étapes suivantes consistant à :
- mettre à disposition des concentrés thrombocytaires se trouvant respectivement dans des poches d'irradiation planes et flexibles, perméables aux UV, obtenus à partir de sang de donneurs et/ou par aphérèse automatique, et se composant d'une multitude d'unités qui peuvent être manipulées individuellement et sont conservées séparément (CT),
- soumettre les CT à une irradiation à la lumière ultraviolette (UV)
**caractérisé en ce que**
- les poches d'irradiation sont remplies à moins de 30 % du volume maximal de remplissage des poches d'irradiation et
- les poches d'irradiation subissent un mouvement pendant l'irradiation avec la lumière UV pour que le contenu des poches d'irradiation soit bien mélangé et que des zones d'épaisseurs de couches changeantes se forment par le biais de ce mouvement, la lumière UV mise en oeuvre comprenant l'irradiation avec une lumière UVC comprise dans la plage allant de 280 nm à 200 nm.

2. Procédé selon une des revendications précédentes, **caractérisé en ce que**, du fait du mouvement, les poches d'irradiation présentent des zones irradiées pour lesquelles il résulte régulièrement des épaisseurs de couches temporairement inférieures à 1 mm, les poches d'irradiation subissant un mouvement par secouage, basculement ou par rotation.

3. Procédé selon une des revendications précédentes, **caractérisé en ce que** le mouvement, en particulier l'amplitude du mouvement, s'effectue de manière à former à l'intérieur des CT des zones irradiées dans lesquelles les épaisseurs de couche sont régulièrement temporairement inférieures à 0,5 mm, les poches d'irradiation subissant un mouvement par secouage, basculement ou par rotation.

4. Procédé selon une des revendications précédentes, **caractérisé en ce que** les poches d'irradiation présentent une face inférieure et une face supérieure et que la somme des aires de la face inférieure et de la face supérieure, qui est en contact ou peut être en contact avec le contenu de la poche, constitue plus de 90 % surfacique, de préférence plus de 99 % surfacique de la surface interne totale du contenu de la poche.

5. Procédé selon une des revendications précédentes, **caractérisé en ce que** l'irradiation comprend également une irradiation avec des UVB compris dans une plage allant de moins de 320 nm à 280 nm et se composant de préférence exclusivement d'une irradiation à des longueurs d'ondes comprises dans une plage allant de moins de 280 nm à 200 nm et de moins de 320 nm à 280 nm.

6. Procédé selon une des revendications précédentes, **caractérisé en ce que** chaque unité est préparée à partir d'échantillons d'au maximum 8 donneurs, de préférence à partir d'au maximum 6 donneurs et, en particulier, chaque unité provient d'un donneur.

7. Procédé selon une des revendications précédentes, **caractérisé en ce que** le CT comprend au moins 1 x 10⁸ thrombocytes par ml, de préférence au moins 5 x 10⁸ thrombocytes par ml.

8. Procédé selon la revendication 5, **caractérisé en ce que** l'irradiation avec les UVB s'effectue à une énergie lumineuse de 0,3 à 5 J/cm², de préférence de 0,5 à 2,5 J/cm².

9. Procédé selon une des revendications précédentes, **caractérisé en ce que** l'irradiation avec les UVC s'effectue à une énergie lumineuse de 0,01 à 2 J/cm², de préférence de 0,1 à 1 J/cm².

10. Procédé selon une des revendications précédentes, **caractérisé en ce que** les CT comprennent
(a) du plasma et un milieu de conservation approprié, où la teneur en plasma est de préférence supérieure à 20 % en poids et/ou
(b) un milieu de conservation aqueux tamponné.

11. Procédé selon une des revendications précédentes, **caractérisé en ce que** les CT sont dépourvus de substances photoactives.

12. Procédé selon une des revendications précédentes, **caractérisé en ce que** les poches d'irradiation sont maintenues par un support de manière à rester mobiles dans l'appareil, dans lequel elles subissent un mouvement et sont irradiées, et ne sont notamment pas compressées entre deux surfaces, par exemple entre des plaques en verre ou en plastique, perméables aux UV.

13. Procédé selon au moins une des revendications précédentes, **caractérisé en ce que** les poches d'irradiation présentent une hauteur de remplissage moyenne de moins de 10 mm, de préférence de moins de 5 mm, et que le mouvement provoque constamment des creux de vague qui présentent des épaisseurs de couches inférieures à la moitié de la hauteur de remplissage moyenne, de préférence des épaisseurs de couches de moins de 1 mm, voire même de moins de 0,1 mm.

14. Procédé selon au moins une des revendications précédentes, **caractérisé en ce que**, pendant l'irradiation, les poches d'irradiation subissent constamment un mouvement avec une amplitude de 0,2 à 8 cm au moins sur l'axe des x et, le cas échéant aussi, sur l'axe des y (l'axe des y étant perpendiculaire à l'axe des x) et **en ce que**, indépendamment de cela, la fréquence de la modification de l'axe du mouvement d'agitation est de 0,5 à 10 Hz.

15. Procédé selon au moins une des revendications précédentes, **caractérisé en ce que**, pendant l'irradiation, les poches d'irradiation sont remplies à au maximum 20 % de leur volume de remplissage maximal.
